# EUROPEAN PATENT APPLICATION

(11) **EP 3 919 390 A1**
(43) Date of publication of application: **08.12.2021**
(21) Application number: 21178076.2
(22) Date of filing: 07.06.2021
(51) Int. Cl.: B64F 1/36, A61L 2/20, B64F 5/30, A61L 9/015, B60H 3/00, F24F 8/26

(54) **OZONE FLASH DISINFECTION SYSTEM FOR AIRCRAFT CABINS**

(30) Priority: 05.06.2020 US 202063035191 P
(71) Applicant: Koninklijke Fabriek Inventum B.V., 3439 MG Nieuwegein (NL)
(72) Inventor: GONZALEZ, Arnau Castillo, 3607AK Maarssen (NL)
(74) Representative: Dehns

(57) **Abstract**

A system comprises an ozone conduit (102) in fluid communication with an aircraft door seal (104) configured to seal the ozone conduit to an aircraft door (106). An ozone source is in fluid communication with the ozone conduit for supplying a flow of ozone gas (O₃) to an aircraft interior through the ozone conduit and aircraft door seal. The ozone conduit can be an ozone inlet line, and an ozone outlet line (116) can be in fluid communication with the aircraft door seal for removal of ozone from the aircraft interior (114).

## Description

### CROSS-REFERENCE TO RELATED APPLICATIONS

This application claims priority to and the benefit of U.S. Provisional Patent Application No. 63/035,191, filed June 5, 2020.

### BACKGROUND

### 1. Field

The present disclosure relates to disinfection such as for combatting pandemics and outbreaks, and more particularly to disinfection for pathogens in for aircraft interiors.

### 2. Description of Related Art

For air travel during outbreaks and pandemics, there are two contamination focus points, namely air and surfaces. For surfaces, various cleaning techniques can be used. These can be labor intensive, time consuming, and/or can involve hazardous chemicals for the crew/passengers/equipment, and nonetheless many such techniques do not guarantee complete disinfection of all surfaces. There is an ongoing need for more effective ways of disinfecting aircraft interiors. This disclosure provides a solution to such needs.

### SUMMARY

A system comprises an ozone conduit in fluid communication with an aircraft door seal configured to seal the ozone conduit to an aircraft door. An ozone source is in fluid communication with the ozone conduit for supplying a flow of ozone gas (O₃) to an aircraft interior through the ozone conduit and aircraft door seal. The ozone conduit can be an ozone inlet line, and an ozone outlet line can be in fluid communication with the aircraft door seal for removal of ozone from the aircraft interior.

An ozone destruction unit can be connected in fluid communication with the aircraft door seal through the ozone outlet line. In embodiments, the ozone destruction unit includes a thermal treatment unit configured to heat ozone gas (O₃) to decompose the ozone gas in to oxygen gas (O₂). In certain embodiments, the ozone destruction unit can include a catalytic converter configured to decompose ozone into oxygen gas (O₂). In embodiments, the catalytic converter can include a carbon decomposition catalyst. The catalytic converter can include a manganese dioxide-based catalyst. In embodiments, an oxygen gas chimney is operatively connected to the catalytic converter for outflowing oxygen gas (O₂) from the ozone destruction unit to an external environment.

A method of disinfecting an aircraft interior can include flushing an aircraft interior with ozone gas (O₃), and after a predetermined residence time of the ozone gas in the aircraft interior, flushing the ozone gas out of the aircraft interior. The method can include sealing an aircraft door seal to a door of the aircraft interior, where flushing the aircraft interior includes flowing ozone gas into the aircraft interior through a conduit sealed to the aircraft interior by the aircraft door seal.

In embodiments, flushing the ozone gas out of the aircraft interior includes flowing ozone gas out of the aircraft interior through an ozone outlet line in fluid communication with the aircraft interior by the aircraft door seal.

In embodiments, the method can further include decomposing ozone gas flushed out of the aircraft interior into oxygen gas (O₂), and releasing the oxygen gas into an ambient environment external to the aircraft interior. In embodiments, flushing the aircraft interior with ozone gas includes flushing the aircraft interior with a mixture of ozone gas and water vapor, wherein the mixture has a relative humidity (RH) above 80%, and wherein the predetermined residence time is greater than or equal to 20 minutes. In certain embodiments, the mixture has an ozone gas concentration of 112 mg/m³.

In certain embodiments, flushing ozone gas out of the aircraft interior includes using action of an Environmental Control System (ECS) to drive the ozone gas out of the aircraft interior. In certain embodiments, flushing ozone gas out of the aircraft interior can include forcing the ozone gas through an ozone gas destruction unit, decomposing the ozone gas (O₃) into oxygen gas (O₂) for greater than or equal to 15 minutes. In certain embodiments, flowing ozone gas into the aircraft interior can include disinfecting 99% of pathogens in the aircraft interior.

These and other features of the systems and methods of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:

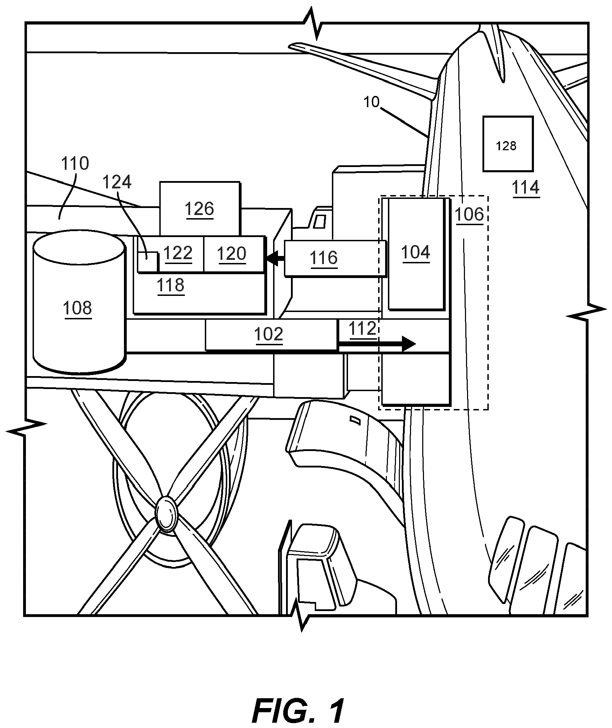

Fig. 1 is a schematic perspective view of an embodiment of a disinfection system constructed in accordance with the present disclosure, showing the disinfection system connected to an aircraft.

### DETAILED DESCRIPTION

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. The systems and methods described herein can be used to efficiently and effectively disinfect an aircraft interior.

Air transport vehicles (e.g. aircraft 10) typically having two main contaminations focus points where pathogens can be spread, through the air and on surfaces. Cleaning surfaces can be labor intensive and a lengthy process as well as requiring the use of chemicals. However, spray and wipe techniques may not ensure complete cleanliness of all surfaces (e.g. in small spaces, crevices, etc.). However, certain gasses, such as ozone, can be capable of killing up to 99.9999% all viruses in a short period of time, without requiring spray and wipe processes.

Ozone is a natural compound, can be easily generated in situ from oxygen or air, and breaks down to oxygen with a half-life of about 20 minutes (± 10 min depending on the environment), for example using an ozone flash disinfection system 100 as described herein. As a gas it can penetrate all areas within an aircraft cabin, including crevices, fixtures, fabrics, and the undersurfaces of furniture, much more efficiently than manual spray and wipe, for example.

The system 100 comprises an ozone conduit 102 in fluid communication with an aircraft door seal 104 configured to seal the ozone conduit 102 to an aircraft door 106. An ozone source 108 (e.g. a tank on-board, a gate service module 110, or an airport utility) is in fluid communication with the ozone conduit 102 for supplying a flow of ozone gas (O₃) 112 to an aircraft interior 114 through the ozone conduit 102 and aircraft door seal 104. The ozone conduit 102 can be an ozone inlet line, and an ozone outlet line 116 can be in fluid communication with the aircraft door seal 104 for removal of ozone from the aircraft interior 114.

An ozone destruction unit 118 can be connected in fluid communication with the aircraft door seal 104 through the ozone outlet line 116. In embodiments, the ozone destruction unit 118 includes a thermal treatment unit 120 configured to heat ozone gas (O₃) to decompose the ozone gas in to oxygen gas (O₂). In certain embodiments, the ozone destruction unit 118 can include a catalytic converter 122 configured to decompose ozone into oxygen gas (O₂). In embodiments, the catalytic converter 122 can include a catalyst 124, for example carbon decomposition and/or a manganese dioxide-based catalyst. In embodiments, an oxygen gas chimney 126 is operatively connected to the catalytic converter 122 for outflowing oxygen gas (O₂) from the ozone destruction unit 118 to an external environment.

A method of disinfecting an aircraft interior (e.g. interior 114) can include flushing the aircraft interior 114 with ozone gas (O₃), and after a predetermined residence time of the ozone gas in the aircraft interior 114, flushing the ozone gas out of the aircraft interior. The method can include sealing an aircraft door seal (e.g. door seal 104) to a door 106 of the aircraft interior 114, where flushing the aircraft interior 114 includes flowing ozone gas into the aircraft interior 114 through a conduit (e.g. conduit 102) sealed to the aircraft interior 114 by the aircraft door seal 104.

In embodiments, flushing the ozone gas out of the aircraft interior 114 includes flowing ozone gas out of the aircraft interior 114 through an ozone outlet line (e.g. outlet line 116) in fluid communication with the aircraft interior 114 by the aircraft door seal 106. In embodiments, the method can further include decomposing ozone gas flushed out of the aircraft interior 114 into oxygen gas (O₂) (e.g. using destruction unit 118) for greater than or equal to 15 minutes, and releasing the oxygen gas into an ambient environment external to the aircraft interior (e.g. via chimney 126). In embodiments, flushing the aircraft interior 114 with ozone gas includes flushing the aircraft interior 114 with a mixture of ozone gas and water vapor, where the mixture has a relative humidity (RH) above 80%, and where the predetermined residence time is greater than or equal to 20 minutes. In certain embodiments, the mixture can have an ozone gas concentration of 112 mg/m³.

In certain embodiments, flushing ozone gas out of the aircraft interior 114 includes using action of an Environmental Control System (ECS) 128 of the aircraft 10 to drive the ozone gas out of the aircraft interior 114. In this manner, the ozone can enter the aircraft interior 114, be taken up by the ECS 128 so that the ozone circulates through the interior 114 via the ECS 128, before passing through an exhaust valve of ECS 128. It is also contemplated that the ECS 128 can be used to help flush latent air within the aircraft interior 114 out of the interior, creating the volume necessary to draw the ozone into the aircraft interior 114 during initial flush. Moreover, passing the ozone through the ECS 128, can disinfect the ECS 128 duct(s) itself. Flowing ozone gas into the aircraft interior 114 can include disinfecting 99% of pathogens in the aircraft interior 114 and/or ECS 128.

The methods and systems of the present disclosure, as described above and shown in the drawings, provide for faster and more effective disinfection of an aircraft interior, for example between flights. While the apparatus and methods of the subject disclosure have been shown and described, those skilled in the art will readily appreciate that changes and/or modifications may be made thereto without departing from the scope of the invention as defined by the claims.

## Claims

1. A system comprising:
an ozone conduit (102) in fluid communication with an aircraft door seal (104) configured to seal the ozone conduit to an aircraft door (106).

2. The system as recited in claim 1, further comprising an ozone source (108) in fluid communication with the ozone conduit for supplying a flow of ozone gas (O₃) to an aircraft interior (114) through the ozone conduit and aircraft door seal.

3. The system as recited in claim 1 or 2, wherein the ozone conduit is an ozone inlet line, and further comprising an ozone outlet line (116) in fluid communication with the aircraft door seal for removal of ozone from the aircraft interior.

4. The system as recited in claim 3, further comprising an ozone destruction unit (118) connected in fluid communication with the aircraft door seal through the ozone outlet line.

5. The system as recited in claim 4, wherein the ozone destruction unit includes a thermal treatment unit (120) configured to heat ozone gas (O₃) to decompose the ozone gas in to oxygen gas (O₂).

6. The system as recited in claim 4, wherein the ozone destruction unit includes a catalytic converter (122) configured to decompose ozone into oxygen gas (O₂).

7. The system as recited in claim 6, wherein the catalytic converter includes a carbon decomposition catalyst (124), or wherein the catalytic converter includes a manganese dioxide-based catalyst.

8. The system as recited in claim 6, further comprising an oxygen gas chimney (126) operatively connected to the catalytic converter for outflowing oxygen gas (O₂) from the ozone destruction unit to an external environment.

9. A method of disinfecting an aircraft interior comprising:
flushing an aircraft interior (114) with ozone gas (O₃);
after a predetermined residence time of the ozone gas in the aircraft interior, flushing the ozone gas out of the aircraft interior.

10. The method as recited in claim 9, further comprising sealing an aircraft door seal (104) to a door (106) of the aircraft interior, wherein flushing the aircraft interior includes flowing ozone gas into the aircraft interior through a conduit sealed to the aircraft interior by the aircraft door seal.

11. The method as recited in claim 10, wherein flushing the ozone gas out of the aircraft interior includes flowing ozone gas out of the aircraft interior through an ozone outlet line (116) in fluid communication with the aircraft interior by the aircraft door seal.

12. The method as recited in claim 9, 10 or 11, further comprising:
decomposing ozone gas flushed out of the aircraft interior into oxygen gas (O₂); and
releasing the oxygen gas into an ambient environment external to the aircraft interior.

13. The method as recited in any of claims 9 to 12, wherein flushing the aircraft interior with ozone gas includes flushing the aircraft interior with a mixture of ozone gas and water vapor, wherein the mixture has a relative humidity, RH, above 80%, and wherein the predetermined residence time is greater than or equal to 20 minutes, and optionally wherein the mixture has an ozone gas concentration of 112 mg/m³.

14. The method as recited in claim 13, wherein flushing ozone gas out of the aircraft interior includes using action of an Environmental Control System, ECS, to drive the ozone gas out of the aircraft interior, or wherein flushing ozone gas out of the aircraft interior includes forcing the ozone gas through an ozone gas destruction unit, decomposing the ozone gas (O₃) into oxygen gas (O₂) for greater than or equal to 15 minutes.

15. The method as recited in any of claims 9 to 14, wherein flowing ozone gas into the aircraft interior includes disinfecting 99% of pathogens in the aircraft interior.
